# EUROPEAN PATENT SPECIFICATION

(11) **EP 0 572 239 B1**
(45) Date of publication and mention of the grant of the patent: **16.10.1996**
(21) Application number: 93304091.7
(22) Date of filing: 26.05.1993
(51) Int. Cl.: C07C 11/02, C07C 7/13

(54) **Separation of hydrocarbon mixtures**
Verfahren zur Auftrennung von Kohlenwasserstoffgemischen
Procédé pour la séparation de mélanges d'hydrocarbures

(30) Priority: 29.05.1992 US 891501; 05.05.1993 US 54641
(43) Date of publication of application: 01.12.1993
(73) Proprietor: THE BOC GROUP, INC., Murray Hill, New Jersey 07974 (US)
(72) Inventor: Ramachandran, Ramakrishnan, Allendale, New Jersey 07401 (US); Dao, Loc, Bound Brook, New Jersey 08805 (US)
(74) Representative: Wickham, Michael

(56) References cited:
- US-A- 2 944 627
- US-A- 3 067 271
- CHEMICAL ABSTRACTS, vol. 80, no. 5, 4 February 1974, Columbus, Ohio, US; abstract no. 26730q, page 382 ;

## Description

The present invention is directed to a method of separating a saturated hydrocarbon from an ethylenically unsaturated hydrocarbon.

It is known to separate saturated hydrocarbons (e.g. propane) from ethylenically unsaturated hydrocarbons (e.g. propylene) by distillation. The mixture of the saturated and unsaturated hydrocarbons is typically obtained from a dehydrogenator or hydrocarbon cracker. The cracker receives a feed gas composed primarily of the saturated hydrocarbon and dehydrogenates or cracks the feed gas to form the ethylenically unsaturated hydrocarbon in an admixture with residual unreacted feed gas.

The conventional method of producing propylene from a feed gas composed primarily of propane usually involves three principal steps:
i) the production of propylene from a propane feed in a dehydrogenator or hydrocarbon cracker.
ii) the separation of the light components, and
iii) the separation of propylene from propane and other heavy components.

The production of propylene from propane is normally carried out in a catalytic dehydrogenation reactor or a thermal cracker. A catalytic dehydrogenator reactor is normally operated at high temperatures (500° to 700°C) and low pressures 20 to 345 kPa (3 to 50 psia. The resulting effluent is cooled and compressed, and the light components such as hydrogen, methane and C₂ hydrocarbons are removed. C₂ components may be removed by a deethanizer; the aforementioned lighter components may be removed by a demethanizer. The heavier components (C₃s and higher) are subsequently fed into a C₃-splitter, which typically is a 2-column distillation system. The first column of the 2-column distillation system separates a substantial portion of the propane to produce a chemical or refinery grade propylene of at least 90 volume percent purity, typically about 96 volume percent. The second column improves the purity level to 99+ volume percent to obtain polymer grade propylene. The heavier components are subsequently removed by a deoiler to reclaim the unreacted propane for recycling back to the reactor.

The process of separating propylene from propane by distillation is both difficult and costly. This is because the production of polymer grade propylene is a very energy-intensive process. Typically, the second distillation column must be nearly equal in size to the first distillation column, adding significant capital expense to the process. Further, the energy required to improve purity from 96 volume percent to 99+ volume percent in a conventional distillation process is more than half of that required to produce chemical or refinery grade purity propylene (about 96 volume percent purity) from a 40 volume percent propane/60 volume percent propylene feed mixture.

In a conventional process for producing propylene from propane, the feed mixture obtained from the reactor at low pressure 20 to 345 kPa (3 to 50 psia) and high temperature (500° to 700°C) must be compressed to higher pressures, typically from 1380 to 4480 kPa (200 to 650 psia), and then cooled to near ambient temperatures (20° to 50°C) in order to remove the light components. Demethanizer and deethanizer columns are normally used for the removal of the light hydrocarbon components. To separate propylene from propane using distillation columns (super-fractionators), the process gas stream may be expanded and cooled further to as low as -50°C.

A propane-rich stream is obtained as the bottom product of the super-fractionator, and this stream is recycled back to the reactor. A propylene-rich gas is obtained from the top of the column as the final product. If propylene purity levels exceeding 96 volume percent are desired, then a second distillation column must be used. Along with the high capital expense of the distillation columns, the increase in pressure and reduction in temperature of the reactor effluent requires a significant consumption of energy which adds to the cost of the system.

Thus, the employment of consecutive distillation columns for the production of an unsaturated hydrocarbon from a feed stream of a saturated hydrocarbon and for the separation of saturated and unsaturated hydrocarbons suffers from two principal disadvantages. The process uses large amounts of energy when the high temperature gas is compressed and cooled prior to entering the distillation system. In addition, a large capital expenditure is incurred when a plurality distillation columns are used to obtain unsaturated hydrocarbon purity levels up to and exceeding 99 volume percent. particularly in the production of propylene from propane.

US-A-4,917,711 describes the ambient temperature adsorption of the unsaturated hydrocarbon from a mixture containing the unsaturated hydrocarbon and a saturated hydrocarbon using a copper adsorbent supported on a high surface area substance.

US-A-3 067 271 discloses a moving bed adsorption process for adsorption of an ethylene-propylene mixture from a gas mixture which additionally incluse alkanes and other components. Desorption is accomplished by a stripping gas. The adsorption is performed at a temperature in the range of about minus 18°C to plus 93°C, usually about 38°C.

The invention aims at providing a separation method which makes it possible to achieve hydrocarbon separation at high product yield and with good selectivity.

According to the present invention there is provided a method of separating a gaseous alkene from a gas mixture comprising of said alkene and one or more alkanes comprising:
(a) subjecting said gas mixture to a first pressure swing adsorption step comprising passing said gas mixture through at least one bed of 4A zeolite adsorbent at a temperature above about 50° C., thereby preferentially adsorbing said alkene from said gas mixture; and
(b) desorbing the adsorbed alkene from said at least one bed.

The present invention is applicable to the separation of alkenes having 2 to 6 carbon atoms from alkanes having 2 to 6 carbon atoms, and is particularly applicable to the separation of one or more alkenes having 2 to 4 carbon atoms from one or more alkanes having 2 to 4 carbon atoms by preferentially adsorbing the alkene(s) in a pressure swing adsorption process carried out alone, as described above, or carried out in combination with a distillation process or another pressure swing adsorption process, the particular system employed depending on the components present in the feed stream being treated and the specification of the final product.

The present invention optionally utilises hybrid process techniques effectively to meet more stringent product specifications. In one hybrid separation method for producing gaseous alkene in accordance with the invention, the gaseous mixture of an alkene and an alkene, such as a mixture of propylene and the corresponding alkane, propane, obtained from the dehydrogenation of a propane feed gas, is compressed and cooled to remove the light components, i.e. hydrocarbons having up to 2 carbon atoms, and then fed to a distillation column for further purification. Since a warm gaseous product is often desired, rather than using two serially-connected distillation columns to separate propane from propylene according to conventional procedures, the second distillation step can be replaced by the method according to the invention.

The bottom product of the distillation column, a saturated hydrocarbon rich stream, is recycled to the reactor. The overhead product, an alkene-rich stream, is heated and sent as a feed to a pressure swing adsorption system for performing the method according to the invention where most of the remaining alkane is removed by the preferential adsorption of the alkene. Any alkane remaining in the pressure swing adsorption unit can be recycled back to the dehydrogenation unit or to the distillation column as a mixture with the non-adsorbed alkene. A high purity alkene vapour (i.e. 99 or higher volume percent alkene) is obtained. Upon regeneration of the adsorbent in the pressure swing adsorption system, an unsaturated hydrocarbon is obtained at purity levels which can exceed 99 volume percent.

If a liquid alkene product is desired, the pressure swing adsorption process is carried out upstream of the distillation step. In this embodiment, the gaseous mixture, which has been stripped of the light hydrocarbon components, is fed into a pressure swing adsorption unit for performing the method according to the invention where a major portion of the alkane is removed and recycled to the reactor. The alkene-rich stream from the pressure swing adsorption unit is then sent to a distillation column where most of the remaining alkane is removed. A liquid alkene product is obtained from the overhead condenser of the distillation column at purity levels which can well exceed 99 volume percent.

In another embodiment of the invention, the light components such as hydrogen, methane, C₂ hydrocarbons and, possibly some higher alkanes from the reactor effluent are removed by a preliminary first pressure swing adsorption process. In this embodiment, the stream exiting the preliminary pressure swing adsorption process is subjected to the above-described pressure swing adsorption process, wherein the alkane is separated and recycled to the reactor and a high purity stream of alkene is obtained as the final product. In this embodiment, very high purity liquid alkene can be obtained by adding a distillation step at the end of the second adsorption process.

Methods according to the present invention will now be described by way of example with reference to the accompanying drawings, in which:
Figure 1 is a schematic view of one embodiment of the invention in which a mixture of saturated and unsaturated hydrocarbons is sent directly to a pressure swing adsorption system;
Figure 2 is a schematic view of another embodiment of the invention, similar to Figure 1, in which a purge gas is used to regenerate the pressure swing adsorption system;
Figure 3 is a schematic view of a further embodiment of the invention in which a first pressure swing adsorption unit treats the light components and a second pressure swing adsorption unit separates the saturated hydrocarbon from the unsaturated hydrocarbon;
Figure 4 is a schematic view of another embodiment of the invention in which the effluent of a dehydrogenation reactor is treated to remove light components in a first pressure swing adsorption unit and a mixture of saturated and unsaturated hydrocarbons is sent to a second pressure swing adsorption unit and the resulting unsaturated hydrocarbon rich feed is cooled, compressed, and sent to a distillation column for further purification;
Figure 5 is a schematic view of another embodiment of the invention which is similar to that shown in Figure 4, wherein the two pressure swing adsorption units are combined into a single pressure swing adsorption system; and
Figure 6 is a schematic view of another embodiment of the invention in which a mixture of saturated and unsaturated hydrocarbons is sent to a distillation column and an unsaturated hydrocarbon rich feed is sent to a pressure swing adsorption system for further purification.

The invention is useful for the separation of gaseous alkenes from gaseous alkanes. The separation is effected by pressure swing adsorption using an adsorbent which more readily adsorbs alkenes than alkanes at the adsorption temperatures employed. Alkenes which can be separated by the process of the invention are generally those having 2 to 6 carbon atoms and include ethylene, propylene, the butenes, the pentenes and the hexenes. The gaseous alkanes from which the alkenes are separated are those generally having 2 to 6 carbon atoms and include ethane, propane, the butanes, the pentanes and the hexanes. In a preferred embodiment the process of the invention is applied to the separation of alkenes having 2 to 4 carbon atoms from alkanes having 2 to 4 carbon atoms. The most beneficial application of the invention involves the separation of an alkene having 2 to 6 carbon atoms from the corresponding alkane, i.e. an alkane having the same number of carbon atoms as the alkene being separated. Preferred separations include ethylene-ethane separation, propylene-propane separation and separations between one of the butenes, e.g i-butylene, and one or more of the butanes, e.g. n-butane or i-butane.

A preferred adsorbent for the adsorption of the alkene from the alkene-alkane mixture is unmodified type 4A zeolite, i.e. the sodium form of type A zeolite, which has a pore size of about 0,4 nm (4 Angstrom). This adsorbent provides enhanced selectivity and capacity in adsorbing alkenes from alkene-alkane mixtures at elevated temperatures.

In some instances it may be desirable to exchange some of the sodium ions by other metal ions. This may be done, provided that the percentage of ions exchanged is not so great that the ability of the adsorbent selectively to adsorb alkenes from alkene-alkane gas mixtures at elevated temperatures is significantly adversely affected. In general, it has been determined that up to about 25 percent of the sodium ions in 4A zeolite can be replaced by ion exchange with other cations without changing the 4A character of the adsorbent. Cations that may be ion exchanged with the 4A zeolite used in the alkene-alkane separation include, among others, potassium, calcium, magnesium, strontium, zinc, cobalt, silver, copper, manganese, cadmium, etc.

4A zeolite which contains certain oxidizable metal cations exhibits enhanced adsorptive capacity and selectivity with respect to the preferential adsorption of alkenes from gaseous alkene-alkane mixtures at temperatures above about 100° C. For instance, copper ion-exchanged 4A zeolite strongly adsorbs ethylene and propylene from mixtures containing these alkenes and the corresponding alkanes at temperatures in the range of about 100 to 200° C.

The temperature at which the adsorption step of the alkene-alkane adsorption process is carried depends upon a number of factors, such as the particular alkene and alkane being separated, the particular adsorbent being used, e.g. unmodified 4A zeolite or a particular metal-exchanged 4A zeolite, and the pressure at which the adsorption is carried out. In general, the adsorption step is carried out at a minimum temperature of about 50° C. and preferably at a temperature of at least about 70° C. The upper temperature limit at which the adsorption step of the process of the invention is carried out is determined by mostly by economics. In general the adsorption step can be carried out at a temperature below the temperature at which the alkene undergoes chemical reaction, such as polymerisation. When unmodified 4A zeolite is used as the adsorbent the reaction is generally carried out at or below 200° C., and is preferably carried out at a temperature at or below 170° C. When certain metal-exchanged 4A zeolites, particularly copper-containing 4A zeolite is used as the adsorbent the adsorption step is generally carried out at temperatures between about 100° C. and 200° C., and is preferably carried out at temperatures in the range of about 125 to 200° C., and is most preferably carried out at temperatures in the range of about 150 to about 200° C.

The pressures at which the adsorption and regeneration steps of the process of the invention are carried out are not critical, and in general, this step can be carried out at any of the usual pressures employed for gas pressure swing adsorption processes. Typically the absolute pressure during the adsorption step will range from about 20 to about 2000 kPa (about 0.2 to about 20) atmospheres absolute), and preferably from about 100 to 1000 kPa (about 1 to 10 atmospheres) and during the regeneration step will range from about 2 to 100 kPa (about 20 millibars to about 1 atmosphere).

To simplify discussion of the invention, the preferred embodiments of the invention will be described with particular reference to propylene as the adsorbed alkene and propane as the nonadsorbed alkane. It should be understood, however, that the present invention is applicable to separation of other gaseous alkenes and alkanes, as detailed above.

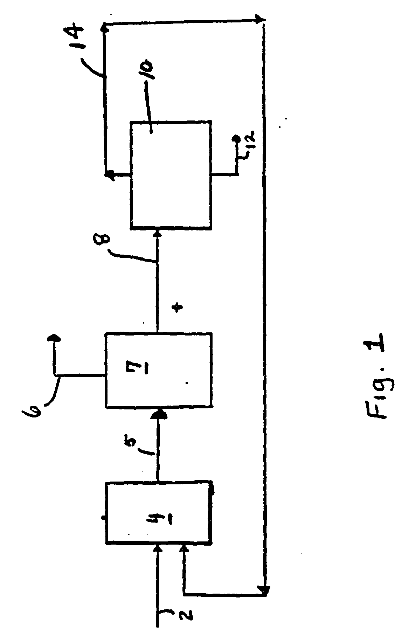

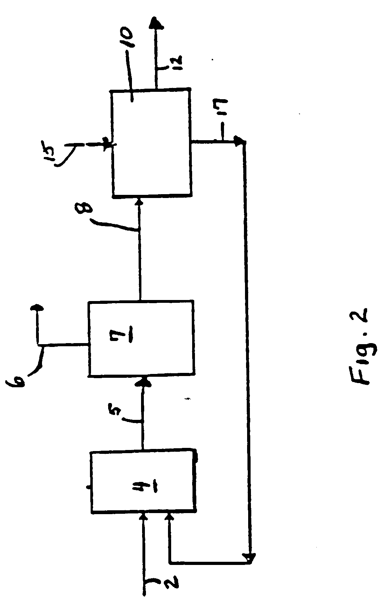

Referring to the drawings and particularly to Figures 1 and 2, there is shown a first embodiment of the invention in which a gaseous mixture of propylene and propane is sent directly to a pressure swing adsorption system. As shown in Figures 1 and 2, a propane feed is sent via line 2 to a propane dehydrogenator or hydrocarbon cracker reactor 4 wherein a portion of the propane (typically about 40 percent by volume) is converted to propylene. The reaction is run under conditions typical in the art including a reaction temperature of 500° to 700°C and a reaction pressure of 20 to 345 kPa (3 to 50 psia). The particular details of the dehydrogenation reaction are well known and form no part of the invention.

The mixture of propane and propylene is sent from dehydrogenator 4 via line 5 to a known device such as a demethanizer and deethanizer distillation column system 7 for removing substantially light components including C₁ and, where appropriate, C₂ hydrocarbons via line 6. The removal of the light components can also be carried out by the known method of compression and cooling.

The propane-propylene mixture, substantially devoid of light components, is sent via line 8 to pressure swing adsorption unit 10. Unit 10 has at least one bed containing 4A type zeolite adsorbent which is adapted to preferentially adsorb propylene while allowing propane to pass through the unit to be recycled with unrecovered propylene to dehydrogenator 4. Details of the construction and operation of pressure swing adsorption systems form no part of this invention, but such systems, including single and multiple beds are disclosed, for example, in US-A-2,944,627.

A substantially pure (99+ volume percent) unsaturated hydrocarbon (propylene) stream exits unit 10 via line 12 to a storage vessel (not shown) or is sent directly to the end user such a polymerisation reactor (not shown).

The propylene adsorbed by the adsorbent is removed through a typical regeneration process employed in pressure swing adsorption systems. The propylene adsorbed in the beds is removed by desorption, preferably under vacuum conditions, with a major portion being recovered through line 12 as product. A minor portion of the propylene is returned with the unreacted propane via line 14 to dehydrogenator 4 for further processing.

The adsorbent beds may be flushed with an inert purge gas such as nitrogen or a portion of the substantially pure unsaturated hydrocarbon product gas as shown specifically in Figure 2. The purge gas mixture is then removed from pressure swing adsorption unit 10. If nitrogen is used as the purge gas, the resulting mixed stream is preferably combusted or vented to the atmosphere. Alternatively, a portion of the propane feed can be used as a purge gas via line 2 through line 15 in which case the off-gas is sent to the dehydrogenator 4. In addition, the product purity can be further improved by repressurising the regenerated adsorbent bed with the unsaturated hydrocarbon product.

The propane feed is reacted in the dehydrogenator 4 at pressures generally in the range of from 20 to 345 kPa (3 to 50 psia) and temperatures of 500° to 700°C. The resulting propane/propylene mixture, after removal of the light components, is sent to pressure swing adsorption unit 10, typically operating at a temperature of 50° to 200°C and an absolute pressure of 100 to 1000 kPa (1 to 10 atmospheres), as noted above. Thus, the operating temperature of pressure swing adsorption unit 10 is generally significantly higher than the operating temperature range of distillation columns in which the gaseous mixture must be cooled to temperatures as low as -50°C. As a consequence, the embodiments of the present invention shown in Figures 1 and 2 are capable of separating saturated and unsaturated hydrocarbons with less cooling duty than is required in known distillation systems.

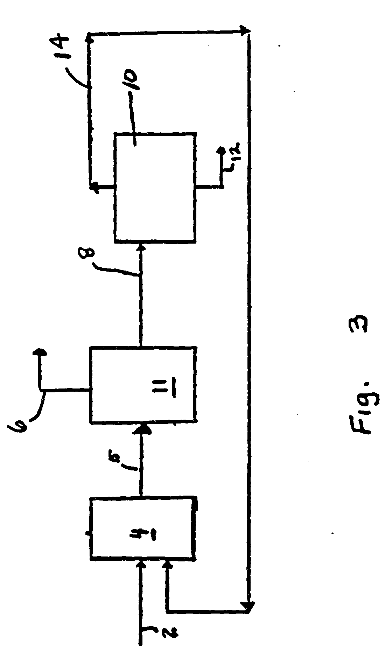

A preliminary pressure swing adsorption unit can be used to remove the light components as an alternative to a demethanizer/ deethanizer distillation system. Referring to Figure 3, the mixture of propane and propylene is introduced into dehydrogenator 4 via line 2, and is discharged therefrom line 5 to preliminary pressure swing adsorption unit 11 which preferentially adsorbs propane and propylene; the light components including hydrogen, methane and C₂ hydrocarbons are consequently removed from the process stream through line 6. The adsorbents which are used in unit 11 to preferentially adsorb the propane and propylene and reject the light components are preferably selected from silica gel and activated carbon. The unreacted propane and the dehydrogenation product (propylene) leave pressure swing adsorption unit 11 via line 8 and enter second pressure swing adsorption unit 10. Alternatively, units 10 and 11 may be combined into a single system with separate beds for recovering the saturated and unsaturated hydrocarbons, and rejecting the light components. The propane-propylene mixture is treated in the same manner as described above in connection with the embodiments of Figures 1 and 2 to provide a substantially pure propylene product.

The embodiments shown and described in connection with Figures 1-3 are particularly adapted for the production of a gaseous unsaturated hydrocarbon such as gaseous propylene. In the embodiments which follow, a distillation column is added to the process scheme to provide options for the production of a liquid alkene product.

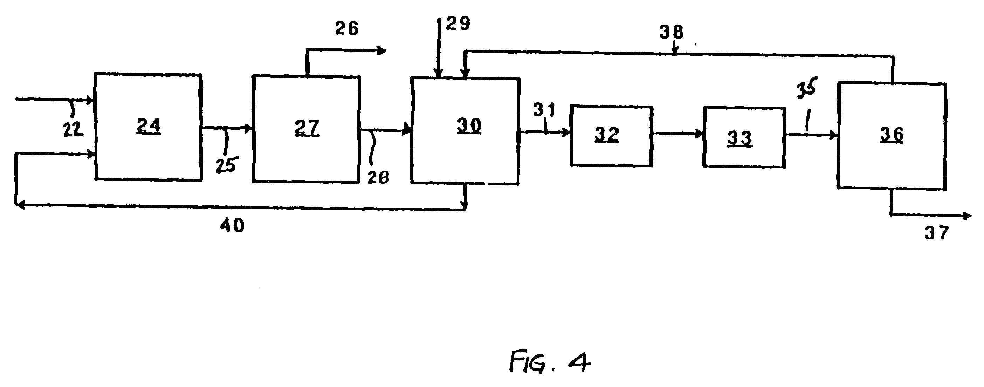

Referring to Figure 4, there is shown another embodiment of the invention in which a pressure swing adsorption system is first used to separate the alkene from a gas mixture to purity levels of about 96 volume percent. The alkene-rich stream is then sent to a distillation column to remove further amounts of slkene and thereby obtain the alkene at purity levels equal to or exceeding 99 volume percent.

The propane feed is sent via line 22 to dehydrogenator or thermal cracker 24 operating at a pressure of 20 to 345 kPa (3 to 50 psia) and a temperature of 500° to 700°C to form a mixture of propane and propylene in a ratio of about 40/60 volume percent as described in the embodiments of Figures 1-3. The mixture of propane and propylene may then be treated to remove the light components. This can be accomplished by compression and cooling, or by means of demethanizer and deethanizer columns, or, as shown specifically in Figure 4, by using pressure swing adsorption unit 27. Unit 27 contains adsorbents such as silica gel and activated carbon which preferentially adsorb propane and propylene. The light components are then removed from unit 27 via line 26.

The gas mixture, now substantially devoid of the light components, proceeds via line 28 to second pressure swing adsorption unit 30 where the initial separation of propylene and propane occurs.

The propylene present in the propylene rich gas obtained from pressure swing adsorption unit 27 is adsorbed in second pressure swing adsorption unit 30, operating at temperature of 50° to 200° C. and an absolute pressure of about 101.3 to 1013 kPa (1 to about 1o atmospheres). The adsorbent beds of unit 30, preferably containing unmodified 4A zeolite, preferentially adsorb propylene to thereby produce a propylene product having a purity level of at least 90 volume percent, typically about 96 volume percent purity.

Pressure swing adsorption unit 30 may be purged with an inert gas such as nitrogen or propylene product gas via line 29. In the former embodiment, the purge gas mixture may be combusted or vented to the atmosphere. In the latter embodiment, the gaseous mixture is preferably recycled to pressure swing adsorption unit 30. The propane feed can be used as a purge gas as well by passing propane through line 29.

The resulting propylene-rich stream is removed from pressure swing adsorption unit 30 via line 31. This stream is compressed, for example, to 10 to 300 psig, by compressor 32 and the resulting compressed stream is cooled in heat exchanger 33 to a temperature of 40° to -50°C. The compressed and cooled gas is then sent via line 35 to distillation column 36. A mixture of propane and propylene is discharged from distillation column 36 and sent as a recycle back to pressure swing adsorption until 30 via line 38. A liquid propylene product having a purity level of 99+ volume percent is obtained as an overhead product via line 37. Since a substantial separation of the propylene and propane has been effected by pressure swing adsorption unit 30, the size of distillation column 36 as well as that of compressor 32 and heat exchanger 33 can be relatively small.

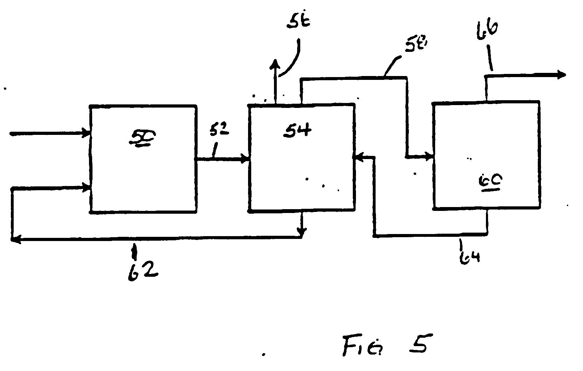

Referring to Figure 5, there is shown an embodiment of the invention in which the pressure swing adsorption unit used to remove the light components from the mixed feed stream is combined vith the pressure swing adsorption unit used to separate the alkene from the alkane to thereby form a consolidated pressure swing adsorption system. The propane-propylene mixed stream obtained from dehydrogenator 50 via line 52 is sent to combined pressure swing adsorption unit 54. Unit 54 has one set of adsorbent beds which preferentially adsorbs propane and propylene and rejects the light components, which are then removed from the system the line 56. The propylene-propane rich mixture, devoid of light components, is sent to a second set of adsorbent beds which preferentially adsorbs rich propylene to purity levels of up to 96 volume percent. The propylene rich stream is sent via line 58 to distillation column 60. Pressure swing adsorption unit 54 is purged to remove propylene and unreacted propane and the resulting gaseous mixture is recycled via line 62 to dehydrogenator 50 or vented to the atmosphere.

A bottom product is obtained from distillation column 60 which is composed primarily of unreacted propane and propylene. This mixed stream is recycled via line 64 to combined pressure swing adsorption unit 54. Substantially pure propylene (99+ volume percent) is removed from distillation column 60 via line 66.

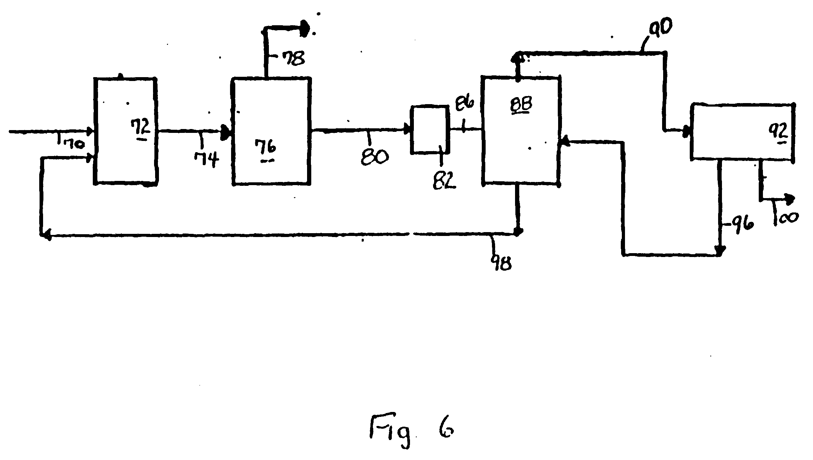

Referring to Figure 6, there is shown another embodiment of the invention in which a distillation column is first used to separate the unsaturated hydrocarbon from a saturated hydrocarbon to purity levels of about 96 volume percent. The unsaturated hydrocarbon rich stream is then sent to a pressure swing adsorption unit to remove further amounts of the alkane and thereby obtain the alkene at purity levels equal to or exceeding 99 volume percent.

The propane feed is sent via line 70 to dehydrogenator or thermal cracker 72 to form a mixture of propane and propylene in a ratio of about 40/60 volume percent as described in connection with the embodiments of Figures 1 and 2. The mixture leaves dehydrogenator 72 via line 74 at a pressure of 20 to 345 kPa (3 to 50 psia) and a temperature of 500° to 700°C. The mixture is then treated to remove light components by cooling and compressing, by the use of demethanizer column 76, as shown specifically in Figure 6 or by a pressure swing adsorption unit, as previously described. The light components are removed via line 78. After exiting demethanizer 76 via line 80, the gaseous mixture is cooled to a temperature of 40° to -50°C in heat exchanger 82.

The cooled hydrocarbon mixture then proceeds via line 86 to distillation column 88 wherein the initial separation of propylene and propane occurs. A propylene-rich stream of at least 90 volume percent, typically about 96 volume percent purity is removed from distillation column 88 via line 90 for entry into pressure swing adsorption unit 92 for further purification. A recycle stream of propane and propylene is also sent from distillation column 88 to dehydrogenator 72 via line 98. A mixture of propane and propylene is recycled to distillation column 88 via line 96.

The propylene present in the propylene rich gas obtained from line 90 is adsorbed in pressure swing adsorption system 92 operating at a temperature of about 50 to about 200°C and an absolute pressure of 101.3 to 1013 kPa (about 1 to about 10 atmospheres). The adsorbent beds, preferably containing unmodified 4A zeolite, preferentially adsorb propylene to thereby produce a propylene product having a purity level of 99+ volume percent which exits the system via line 100.

The adsorbent beds of the pressure swing adsorption system may be regenerated as described in connection with the description of the embodiment illustrated in Figures 1 and 2. An inert purge gas may also be provided. The purge gas is preferably a non-adsorbing gas such as methane or propane. When a purge gas is used the off gas will be sent directly to dehydrogenator 72. In the absence of a purge gas, a vacuum pump (not shown) may be used in a customary manner.

The invention is further illustrated in the following examples wherein, unless otherwise indicated, parts, percentages and ratios are on a volume basis.

### EXAMPLES 1-8

These examples were carried out in a laboratory pressure swing adsorption apparatus comprising a pair of parallel-arranged stainless steel adsorption vessels each equipped with a heating jacket and containing 3842 grams (about 2.5 litres) of the indicated 4A type zeolite. The adsorption cycle consisted of the steps: bed equalisation (9 secs.), countercurrent repressurisation with nonadsorbed product (7 secs.), cocurrent adsorption of a feed gas (34 secs.), bed equalisation (9 secs.), and countercurrent depressurisation (41 secs.). The total time for a half-cycle was 50 secs.
Each experiment was carried out for a minimum period of three hours, which ensured the existence of steady state conditions. The adsorption was carried out at a pressure of about 170 kPa (about 25 psia), the beds were equalised to a pressure of 70 kPa (10 psia), and the beds were evacuated to an absolute pressure of 10 to 20 kPa (100 to 200 millibars). In the examples a feed gas composed of about 12% propane and 88% propylene (by volume) was used as the feed gas. The flow rate of the feed stream, high pressure product (HPP) and low pressure product (LPP), which are determined for standard conditions, i.e. room temperature and atmospheric pressure, are reported in standard litres per minute (SLPM).

**TABLE**

| | | | Flow Rate | | | Propylene Recovered % | Propane Rejected % |
|---|---|---|---|---|---|---|---|
| Ex. | Adsorbent | Temp. °C | Feed | HPP | LPP | | |
| 1 | 4A | 30 | 3.37 | 0.29 | 3.08 | 96.0 | 43.7 |
| 2 | 4A | 70 | 5.59 | 0.56 | 5.03 | 96.3 | 66.6 |
| 3 | 4A | 90 | 6.13 | 0.58 | 5.55 | 97.4 | 70.9 |
| 4 | 4A | 110 | 5.94 | 0.52 | 5.43 | 98.2 | 71.0 |
| 5 | 4A | 175 | 9.88 | 1.78 | 8.10 | 91.0 | 82.2 |
| 6 | 5A | 30 | 8.21 | 1.48 | 6.73 | 85.7 | 45.0 |
| 7 | 5A | 110 | 11.58 | 1.40 | 10.18 | 90.4 | 28.8 |
| 8 | 13X | 90 | 10.28 | 1.47 | 10.28 | 87.6 | 28.5 |

The above examples illustrate the benefits obtained by the invention. In the above series of examples, Examples 2-5 fall within the scope of the invention and Examples 1 and 6-8 are comparative examples. Examples 2-5 illustrate that when experiments within the scope of the invention were conducted a high percentage of the propylene in the feed stream was recovered, and good propane rejection rates were obtained. In comparison to this, Example 1 illustrates that when the adsorption process is practiced at 30° C., a high percentage of the propylene in the feed stream was recovered but only 43.7 percent of the propane in the feed stream was rejected (i.e. was in the nonadsorbed product stream).

Comparative Examples 6-8 show that 5A and 13X zeolites are much inferior to 4A zeolite for high temperature adsorptive separation of propylene and propane in that very poor propane rejection resulted from the use of these adsorbents.

## Claims

1. A method of separating a gaseous alkene from a gas mixture comprising of said alkene and one or more alkanes comprising:
(a) subjecting said gas mixture to a first pressure swing adsorption step comprising passing said gas mixture through at least one bed of 4A zeolite adsorbent at a temperature above 50° C., thereby preferentially adsorbing said alkene from said gas mixture; and
(b) desorbing the adsorbed alkene from said at least one bed.

2. A method as claimed in claim 1, in which the 4A zeolite adsorbent is unmodified.

3. A method as claimed in any one of the preceding claims in which the adsorption takes place at a temperature between 50°C and 200°C.

4. A method as claimed in any one of the preceding claims, in which the alkane contains from 2 to 4 carbon atoms and the alkene contains the same number of carbon atcms as the alkane.

5. A method as claimed in any one of the preceding claims, wherein said gas mixture comprises either propylene and propane or ethylene and ethane.

6. A method as claimed in any one of the preceding claims, wherein said gas mixture is passed through said bed of 4A zeolite adsorbent temperature in the range of 70 to 170°C and an absolute pressure of 100 to 1000 kPa.

7. A method as claimed in any one of the preceding claims, further comprising removing light components comprising hydrogen and alkanes having fewer carbon atoms than said alkene from said gas mixture upstream of subjecting said gas mixture to said first pressure swing adsorption process.

8. A method as claimed in Claim 7, wherein the step of removing said light components from said gas mixture comprises subjecting said gas mixture to a preliminary pressure swing adsorption process prior to said first pressure swing adsorption process, thereby preferentially adsorbing said alkene and alkanes other than those having fewer carbon atoms than said alkene from said gas mixture.

9. A method as claimed in any one of claims 1 to 7, further comprising fractionating the desorbate from said first pressure swing adsorption process in a distillation column and removing a high purity alkene fraction as an overhead stream from said distillation column.

10. A method as claimed in Claim 8, further comprising cooling the desorbate from said preliminary pressure swing adsorption process and distilling the cooled desorbate, thereby producing as the feed stream to said first pressure swing adsorption process a gas mixture concentrated in said alkene.

11. A method as claimed in Claim 10, further comprising the steps of dehydrogenating a feed stream comprising an alkane to form said gas mixture and recycling unreacted alkane from said first pressure swing adsorption system to the unit in which said step of dehydrogenating the feed stream is carried out.

12. A method as claimed in Claim 10, further comprising the steps of dehydrogenating a feed stream comprised substantially of an alkane to form said gas mixture, recycling unreacted alkane from said first pressure swing adsorption process to the distillation column and recycling unreacted alkane from the distillation column to the unit in which said step of dehydrogenating the feed stream is carried out.

## Patentansprüche

1. Verfahren zum Abtrennen eines gasförmigen Alkens von einem Gasgemisch, das das Alken und eines oder mehrere Alkane enthält, umfassend:
(a) Unterwerfen des Gasgemisches unter einen ersten Druckschwingungsadsorptionsschritt, der das Hindurchführen des Gasgemisches durch mindestens ein Bett aus einem 4A-Zeolith-Adsorptionsmittel bei einer Temperatur oberhalb von 50 °C umfaßt, wodurch vorzugsweise das Alken aus dem Gasgemisch adsorbiert wird; und
(b) Desorbieren des adsorbierten Alkens von mindestens einem Bett.

2. Verfahren nach Anspruch 1, wobei das 4A-Zeolith-Adsorptionsmittel nicht modifiziert ist.

3. Verfahren nach einem der vorstehenden Ansprüche, wobei die Adsorption bei einer Temperatur zwischen 50 ° und 200 °C stattfindet.

4. Verfahren nach einem der vorstehenden Ansprüche, wobei das Alkan 2 bis 4 Kohlenstoffatome und das Alken die gleiche Anzahl von Kohlenstoffatomen enthält wie das Alkan.

5. Verfahren nach einem der vorstehenden Ansprüche, wobei das Gasgemisch entweder Propylen und Propan oder Ethylen und Ethan enthält.

6. Verfahren nach einem der vorstehenden Ansprüche, wobei das Gasgemisch bei einer Temperatur im Bereich von 70 ° bis 170 °C und einem absoluten Druck von 100 bis 1000 kPa durch das Bett aus einem 4A-Zeolith-Adsorptionsmittel geführt wird.

7. Verfahren nach einem der vorstehenden Ansprüche, das weiter das Entfernen von leichten Komponenten, die Wasserstoff und Alkane mit weniger Kohlenstoffatomen als das Alken umfassen, aus dem Gasgemisch, stromaufwärts von dem Ort umfaßt, an dem das Gasgemisch dem ersten Druckschwingungsadsorptionsverfahren unterzogen wird.

8. Verfahren nach Anspruch 7, wobei der Schritt, die leichten Komponenten aus dem Gasgemisch zu entfernen, das Unterwerfen des Gasgemisches unter ein vorhergehendes Druckschwingungsadsorptionsverfahren vor dem ersten Druckschwingungsadsorptionsverfahren umfaßt, wodurch vorzugsweise das Alken und andere Alkane als diejenigen adsorbiert werden, die weniger Kohlenstoffatome aufweisen als das Alken aus dem Gasgemisch.

9. Verfahren nach einem der Ansprüche 1 bis 7, das außerdem das Fraktionieren des Desorbats aus dem ersten Druckschwingungsadsorptionsverfahren in einer Destillationssäule und das Entfernen einer Alkenfraktion hoher Reinheit als Kopfproduktstrom aus der Destillationssäule umfaßt.

10. Verfahren nach Anspruch 8, das weiter das Kühlen des Desorbats aus dem vorhergehenden Druckschwingungsadsorptionsverfahren und das Destillieren des abgekühlten Desorbats umfaßt, wodurch ein Gasgemisch als Speisestrom des ersten Druckschwingungsadsorptionsverfahrens hergestellt wird, worin das Alken konzentriert ist.

11. Verfahren nach Anspruch 10, das weiter die Schritte umfaßt, einen ein Alkan umfassenden Speisestrom zu dehydrieren, um das Gasgemisch zu bilden, und nicht umgesetztes Alkan aus dem ersten Druckschwingungsadsorptionssystem in die Einheit zurückzuführen, in der der Schritt durchgeführt wird, den Speisestrom zu dehydrieren.

12. Verfahren nach Anspruch 10, das weiter die Schritte umfaßt, einen im wesentlichen aus einem Alkan bestehenden Speisestrom zu dehydrieren, wodurch das Gasgemisch gebildet wird, nicht umgesetztes Alkan aus dem ersten Druckschwingungsadsorptionsverfahren in die Destillationssäule zurückzuführen und nicht umgesetztes Alkan aus der Destillationssäule in die Einheit zurückzuführen, in der der Schritt durchgeführt wird, den Speisestrom zu dehydrieren.

## Revendications

1. Procédé de séparation d'un alcène gazeux d'un mélange de gaz contenant l'alcène et un ou plusieurs alcanes, comprenant :
(a) le traitement du mélange de gaz dans une première étape d'adsorption avec compression alternée, comprenant la circulation du mélange de gaz dans un lit au moins d'un adsorbant de zéolite 4A à une température supérieure à 50 °C, de manière que l'alcène du mélange de gaz subisse une adsorption préférentielle, et
(b) la désorption de l'alcène adsorbé dudit lit au moins.

2. Procédé selon la revendication 1, dans lequel l'adsorbant de zéolite 4A est d'un type non modifié.

3. Procédé selon l'une quelconque des revendications précédentes, dans lequel l'adsorption est réalisée à une température comprise entre 50 °C et 200 °C.

4. Procédé selon l'une quelconque des revendications précédentes, dans lequel l'alcane contient 2 à 4 atomes de carbone et l'alcène contient le même nombre d'atomes de carbone que l'alcane.

5. Procédé selon l'une quelconque des revendications précédentes, dans lequel le mélange de gaz contient du propylène et du propane ou de l'éthylène et de l'éthane.

6. Procédé selon l'une quelconque des revendications précédentes, dans lequel le mélange gazeux circule dans le lit de l'adsorbant de zéolite 4A à une température comprise entre 70 et 170 °C et à une pression absolue comprise entre 100 et 1 000 kPa.

7. Procédé selon l'une quelconque des revendications précédentes, comprenant en outre l'extraction des éléments constituants légers contenant l'hydrogène et les alcanes ayant un plus petit nombre d'atomes de carbone que l'alcène, dans le mélange gazeux qui se trouve en amont de l'opération de traitement du mélange de gaz par la première opération d'adsorption par compression alternée.

8. Procédé selon la revendication 7, dans lequel l'étape d'extraction des éléments constituants légers du mélange de gaz comprend le traitement du mélange de gaz dans une opération préliminaire d'adsorption par compression alternée avant la première opération d'adsorption par compression alternée, si bien que l'alcène et les alcanes autres que ceux qui ont un plus petit nombre d'atomes de carbone que l'alcène sont adsorbés préférentiellement et ainsi séparés du mélange de gaz.

9. Procédé selon l'une quelconque des revendications 1 à 7, comprenant en outre le fractionnement de la matière désorbée de la première opération d'adsorption par compression alternée dans une colonne de distillation, et l'extraction de la fraction d'alcène de pureté élevée comme courant de tête de la colonne de distillation.

10. Procédé selon la revendication 8, comprenant en outre le refroidissement de la matière désorbée de l'opération d'adsorption préliminaire par compression alternée et la distillation de la matière désorbée et refroidie, pour la formation de cette manière, comme courant d'alimentation de la première opération d'adsorption par compression alternée, d'un mélange de gaz concentré en alcène.

11. Procédé selon la revendication 10, comprenant en outre des étapes de déshydrogénation d'un courant d'alimentation contenant un alcane pour la formation du mélange de gaz, et de recyclage de l'alcane qui n'a pas réagi provenant du premier circuit d'adsorption par compression alternée vers l'ensemble dans lequel est exécutée l'étape de déshydrogénation du courant d'alimentation.

12. Procédé selon la revendication 10, comprenant en outre des étapes de déshydrogénation d'un courant d'alimentation essentiellement formé d'un alcane pour la constitution du mélange de gaz, de recyclage de l'alcane qui n'a pas réagi, provenant de la première opération d'adsorption par compression alternée vers la colonne de distillation, et de recyclage de l'alcane qui n'a pas réagi, provenant de la colonne de distillation, vers l'ensemble dans lequel est réalisée l'étape de déshydrogénation du courant d'alimentation.
